# EUROPEAN PATENT SPECIFICATION

(11) **EP 3 203 240 B1**
(45) Date of publication and mention of the grant of the patent: **03.10.2018**
(21) Application number: 17154501.5
(22) Date of filing: 03.02.2017
(51) Int. Cl.: G01N 33/68

(54) **METHOD FOR DETERMINING ACUTE MYOCARDIAL INFARCTION**
VERFAHREN ZUR BESTIMMUNG AKUTER MYOKARDINFARKTE
PROCÉDÉ DE DÉTERMINATION DE L'INFARCTUS AIGU DU MYOCARDE

(30) Priority: 04.02.2016 JP 2016019519
(43) Date of publication of application: 09.08.2017
(73) Proprietor: Matsumori, Akira, Minoh-shi, Osaka 562-0005 (JP)
(72) Inventor: Matsumori, Akira, Minoh-shi, Osaka 562-0005 (JP)
(74) Representative: Paemen, Liesbet R.J.

(56) References cited:
- WO-A1-2006/040409
- WO-A2-2015/185672
- US-A1- 2003 113 728
- S. JACQUET ET AL: "Identification of Cardiac Myosin-binding Protein C as a Candidate Biomarker of Myocardial Infarction by Proteomics Analysis", MOLECULAR & CELLULAR PROTEOMICS, vol. 8, no. 12, 1 December 2009 (2009-12-01), pages 2687-2699, XP055358922, US ISSN: 1535-9476, DOI: 10.1074/mcp.M900176-MCP200
- Angela Tavares ET AL: "Early loss of cardiac function in acute myocardial infarction is associated with redox imbalance", Experimental and clinical cardiology, 1 January 2012 (2012-01-01), page 263, XP055358938, Canada Retrieved from the Internet: URL:https://www.ncbi.nlm.nih.gov/pmc/artic les/PMC3627290/pdf/ecc17263.pdf

### SEQUENCE LISTING

<110> Matsumori, Akira
<120> Determination method of acute myocardial infarction
<130> MTS-002-EP
<150> JP2016-019519
   <151> 2016-02-04
<160> 2
<170> PatentIn version 3.5
<210> 1
   <211> 224
   <212> PRT
   <213> Homo sapiens
<400> 1
   Met Pro Gly Gly Leu Leu Leu Gly Asp Val Ala Pro Asn Phe Glu Ala
   1               5                   10                  15
   Asn Thr Thr Val Gly Arg Ile Arg Phe His Asp Phe Leu Gly Asp Ser
               20                  25                  30
   Trp Gly Ile Leu Phe Ser His Pro Arg Asp Phe Thr Pro Val Cys Thr
           35                  40                  45
   Thr Glu Leu Gly Arg Ala Ala Lys Leu Ala Pro Glu Phe Ala Lys Arg
       50                  55                  60
   Asn Val Lys Leu Ile Ala Leu Ser Ile Asp Ser Val Glu Asp His Leu
   65                  70                  75                  80
   Ala Trp Ser Lys Asp Ile Asn Ala Tyr Asn Cys Glu Glu Pro Thr Glu
                   85                  90                  95
   Lys Leu Pro Phe Pro Ile Ile Asp Asp Arg Asn Arg Glu Leu Ala Ile
               100                 105                 110
   Leu Leu Gly Met Leu Asp Pro Ala Glu Lys Asp Glu Lys Gly Met Pro
           115                 120                 125
   Val Thr Ala Arg Val Val Phe Val Phe Gly Pro Asp Lys Lys Leu Lys
       130                 135                 140
   Leu Ser Ile Leu Tyr Pro Ala Thr Thr Gly Arg Asn Phe Asp Glu Ile
   145                 150                 155                 160
   Leu Arg Val Val Ile Ser Leu Gln Leu Thr Ala Glu Lys Arg Val Ala
                   165                 170                 175
   Thr Pro Val Asp Trp Lys Asp Gly Asp Ser Val Met Val Leu Pro Thr
               180                 185                 190
   Ile Pro Glu Glu Glu Ala Lys Lys Leu Phe Pro Lys Gly Val Phe Thr
           195                 200                 205
   Lys Glu Leu Pro Ser Gly Lys Lys Tyr Leu Arg Tyr Thr Pro Gln Pro
       210                 215                 220
<210> 2
   <211> 1715
   <212> RNA
   <213> Homo sapiens
<400> 2
   gaaccaaccg guugcuugcu gucccagcgg cgcccccuca ucaccgucgc caugcccgga 60
   ggucugcuuc ucggggacgu ggcucccaac uuugaggcca auaccaccgu cggccgcauc 120
   cguuuccacg acuuucuggg agacucaugg ggcauucucu ucucccaccc ucgggacuuu 180
   accccagugu gcaccacaga gcuuggcaga gcugcaaagc uggcaccaga auuugccaag 240
   aggaauguua aguugauugc ccuuucaaua gacaguguug aggaccaucu ugccuggagc 300
   aaggauauca augcuuacaa uugugaagag cccacagaaa aguuaccuuu ucccaucauc 360
   gaugauagga aucgggagcu ugccauccug uugggcaugc uggauccagc agagaaggau 420
   gaaaagggca ugccugugac agcucgugug guguuuguuu uugguccuga uaagaagcug 480
   aagcugucua uccucuaccc agcuaccacu ggcaggaacu uugaugagau ucucagggua 540
   gucaucucuc uccagcugac agcagaaaaa aggguugcca ccccaguuga uuggaaggau 600
   ggggauagug ugaugguccu uccaaccauc ccugaagaag aagccaaaaa acuuuucccg 660
   aaaggagucu ucaccaaaga gcucccaucu ggcaagaaau accuccgcua cacaccccag 720
   ccuuaagucu cuuggagaag cuggugcugu gagccagagg augucagcug ccaauugugu 780
   uuuccugcag caauuccaua aacacauccu ggugucauca cagccaaggu uuuuagguug 840
   cuauaccaau ggcuuauuaa augaaaaugg cacuaaaagu uucuugagau ucuuuauacu 900
   cucugccuuc agcaaucaau uccauucaua caucagcacu cugcugguuc uguuugaaau 960
   auguucugua uuuaaaacuc aaaucuuguu ggaucucugc agggcuugug accaaugaag 1020
   ucauauuugu ugaugguuga caaagcuugc uucacuccau cagagaauga cuaucaauuu 1080
   uuuuuuaacu guccuaucac guccucuccu gucacccauu uugaagagug gcagaacuug 1140
   aaguucaacu uccucuguaa auauccaagu auaaagccca ggaacuucua gaauaaccca 1200
   gaugcgcuuu aauuuuuuuu aauauguuuu gaucacagaa cuucuagaau aacccagaug 1260
   cucuuucaua uucuuuuaau acaucuugau cacagcuggg ggaaaaaaag cuuuuuaauu 1320
   cuauaccuuc cuaguagaua agugaagagc agggaaagag accuuuaaau auuuugcuau 1380
   aaaaaaauuu gugauaaguu ucuaucaaaa uggggagauu gcagaaaagg cuucccuugg 1440
   cucccaagga gguguagcag gugugagcaa uauuagugcc augugccuuu cacacagggu 1500
   uugcauuuau cagucuguuu uccgaugaug uguacaugaa agaguacacc augugaagag 1560
   aagagagaau gauugaaaau guuuuaguau agaacucuuc uugcaguggg uugcuauuuu 1620
   cuagauuuua cuuuuuaggg aacaaaauaa aauccuuugu uaaaacuggg aaaaaaaaaa 1680
   aaaaaaaaaa aaaaaaaaaa aaaaaaaaaa aaaaa 1715

## Description

### TECHNICAL FIELD

The present disclosure relates to a method for supporting a diagnosis of acute myocardial infarction, a device for supporting a diagnosis of acute myocardial infarction, and a computer program for supporting a diagnosis of acute myocardial infarction.

### BACKGROUND

Troponin is known as an acute myocardial infarction marker. Non-Patent Document 1 describes that troponin starts to increase in 2 or 3 hours after the death of cardiomyocytes, peaks after 24 hours, and is in a known state for 1 to 2 weeks (Patil, et al. (2011) Clin. Cardiol. 34, 12, 723-728).

### SUMMARY OF THE INVENTION

Acute myocardial infarction is subjected to reperfusion therapy within 12 hours and more preferably 6 hours after the onset, whereby the mortality rate can be lowered without increasing a necrotic range of the heart muscle. Therefore, it is desirable to detect the presence or absence of the onset of acute myocardial infarction as soon as possible after being taken to hospital. However, when being taken to hospital, patients often lose consciousness, and thus there are many cases where it is difficult to ask patients detailed questions about the presence or absence of a symptom of heart disease such as chest pain. There is also a case where a patient does not feel a symptom of heart disease before the onset of acute myocardial infarction. Also in electrocardiography, ST increase, appearance of abnormal Q wave and the like are observed at the onset of acute myocardial infarction. However, these are not necessarily said to be phenomena specific to acute myocardial infarction.

Based on these problems, an object of the present invention is to provide a novel marker having a high specificity to acute myocardial infarction, and a method for supporting a diagnosis of acute myocardial infarction, on the basis of a measured value of the novel marker.

The present inventor has intensively studied, and found that the measured value of peroxiredoxin in a biological sample of a patient with acute myocardial infarction is significantly high as compared with the value of a healthy subject and other patients with heart disease, and completed the present invention.

The present invention has been completed based on the above knowledge, and is defined in the appended claims. Following embodiments are disclosed.
Item 1. A method for supporting a diagnosis of acute myocardial infarction comprising:
   (a) a step of obtaining a measured value of peroxiredoxin in a biological sample collected from a test subject, and
   (b) a step of obtaining information on acute myocardial infarction based on the obtained measured value of peroxiredoxin.
Item 2. The method according to claim 1, wherein the biological sample is a biological sample collected within 2 hours after the test subject develops a symptom of heart disease.
Item 3. The method according to claim 1 or 2, wherein the biological sample is a blood sample.
Item 4. The method according to any one of claims 1 to 3, wherein,
   the measured value of peroxiredoxin and a predetermined threshold are compared in the step (b), and
   when the measured value is the threshold or more, information showing that the test subject has acute myocardial infarction is obtained.
Item 5. The method according to any one of claims 1 to 3, wherein,
   the measured value of peroxiredoxin and a predetermined threshold are compared in the step (b), and
   when the measured value is less than the threshold, information showing that the test subject does not have acute myocardial infarction is obtained.
Item 6. The method according to any one of claims 1 to 5, wherein the measured value of peroxiredoxin is an expression level of a peroxiredoxin gene or a measured value of a peroxiredoxin protein.
Item 7. Use of a reagent for the method for supporting a diagnosis of acute myocardial infarction according to any one of claims 1 to 6, wherein
   the reagent comprises an anti-peroxiredoxin antibody.
Item 8. Use of a kit for the method for supporting a diagnosis of acute myocardial infarction according to any one of claims 1 to 6, the kit comprising:
   a first anti-peroxiredoxin antibody;
   a solid phase;
   a second anti-peroxiredoxin antibody and a labeling substance.
Item 9. A device for supporting a diagnosis of acute myocardial infarction, equipped with a computer comprising a processor and a memory under the control of the processor, wherein
   a computer program for making the computer execute
   (a) a step of obtaining a measured value of peroxiredoxin in a biological sample of a test subject and
   (b) a step of outputting information on acute myocardial infarction based on the measured value, is recorded in the memory.
Item 10. The device for supporting a diagnosis of acute myocardial infarction according to claim 9, wherein the computer is configured to execute operations comprising:
   a step of comparing the measured value and a predetermined threshold in the step (b),
   a step of determining that the test subject has acute myocardial infarction when the measured value is the threshold or more, and
   a step of outputting information showing that the test subject has acute myocardial infarction are executed.
Item 11. The device for supporting a diagnosis of acute myocardial infarction according to claim 9, wherein the computer is configured to execute operations comprising:
   a step of comparing the measured value and a predetermined threshold in the step (b),
   a step of determining that the test subject does not have acute myocardial infarction when the measured value is less than the threshold, and
   a step of outputting information showing that the test subject does not have acute myocardial infarction are executed.
Item 12. The device according to any one of claims 9 to 11, wherein the measured value of peroxiredoxin is an expression level of a peroxiredoxin gene or a measured value of a peroxiredoxin protein.
Item 13. A computer program for making a computer comprising a processor and a memory under the control of the processor execute operations comprising:
   (a) a step of obtaining a measured value of peroxiredoxin in a biological sample of a test subject; and
   (b) a step of outputting information on acute myocardial infarction based on the measured value.
Item 14. The computer program according to claim 13, wherein the processor is configured to execute operations comprising:
   a step of comparing the measured value and a predetermined threshold in the step (b);
   a step of determining that the test subject has acute myocardial infarction when the measured value is the threshold or more; and
   a step of outputting information showing that the test subject has acute myocardial infarction are executed.
Item 15. The computer program according to claim 13, wherein the processor is configured to execute operations comprising:
   a step of comparing the measured value and a predetermined threshold in the step (b);
   a step of determining that the test subject does not have acute myocardial infarction when the measured value is less than the threshold; and
   a step of outputting information showing that the test subject does not have acute myocardial infarction are executed.

The present invention can provide a method for supporting a diagnosis of acute myocardial infarction.

### BRIEF DESCRIPTION OF THE DRAWINGS

Fig. 1 is a schematic view showing an example of an inspection kit.
Fig. 2 is a schematic view showing an example of a diagnosis supporting device.
Fig. 3 is a block diagram showing a hardware configuration of a diagnosis supporting device.
Fig. 4 is a flow chart of a method for detecting a heart failure patient using a diagnosis supporting device.
Fig. 5 shows a scatter diagram of a peroxiredoxin 6 concentration in the serum of each group.

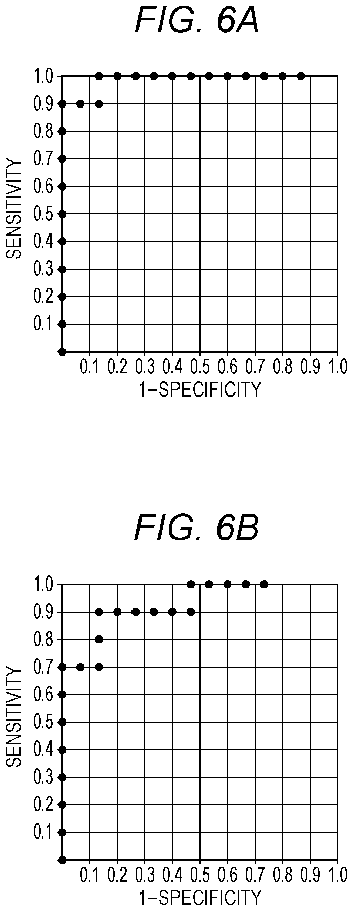

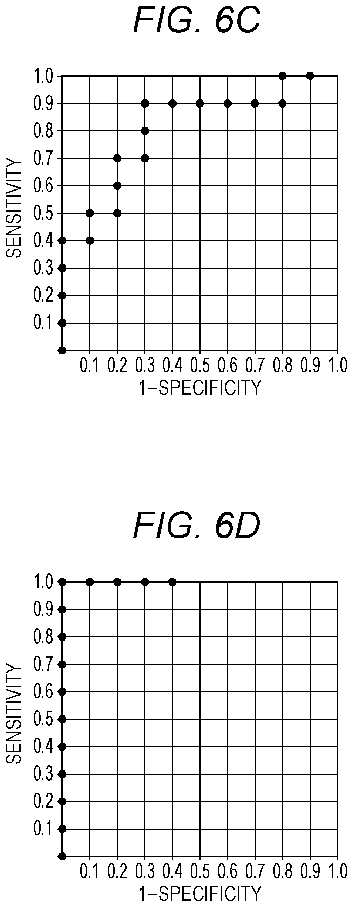

Fig. 6A shows an ROC curve of the measured value of peroxiredoxin 6 of an acute myocardial infarction patient group and the measured value of peroxiredoxin 6 of a dilated cardiomyopathy patient group. Fig. 6B shows an ROC curve of the measured value of peroxiredoxin 6 of an acute myocardial infarction patient group and the measured value of peroxiredoxin 6 of an ischemic heart failure patient group. Fig. 6C shows an ROC curve of the measured value of peroxiredoxin 6 of an acute myocardial infarction patient group and the measured value of peroxiredoxin 6 of an atrial fibrillation patient group. Fig. 6D shows an ROC curve of the measured value of peroxiredoxin 6 of an acute myocardial infarction patient group and the measured value of peroxiredoxin 6 of a healthy subject group.

### DETAILED DESCRIPTION OF THE PREFERRED EMBODIMENTS

### 1. Explanation of terms

First, the terms used in the present specification, claims and abstract will be explained.

In the present disclosure, the term "acute myocardial infarction" refers to a blocking due to embolus, thrombus or the like of vasa vasorum of the heart (coronary artery, its branches and the like). Necrosis of the myocardial cells associated with blocking may occur or may not occur. In acute myocardial infarction, a symptom of heart disease may appear or may not appear, before blocking of vasa vasorum of the heart.

In the present disclosure, the "symptom of heart disease" includes palpitation, shortness of breath, fatigability, vertigo, nausea, chest pain, loss of consciousness, and the like. The "symptom of heart disease" also includes electrocardiographic abnormalities and the like. The "symptom of heart disease" is preferably vertigo, nausea, chest pain, loss of consciousness, and the like.

In the present disclosure, the "individual" is not particularly limited, but the individual includes humans and mammals other than humans. Examples of mammals other than humans include cows, horses, sheep, goats, pigs, dogs, cats, rabbits, monkeys, and the like. The individual is preferably human. Age and sex of the individual do not matter.

The "test subject" may be an individual having a history of acute myocardial infarction and other heart diseases or an individual having no history of acute myocardial infarction and other heart diseases. The test subject may be an individual having subjective symptoms such as palpitation, pain or discomfort in the chest, stagger or general malaise, or an individual having no symptoms. The test subject also includes a specimen suspected of having acute myocardial infarction, in accordance with known diagnosis criteria such as physiological tests such as medical interview, electrocardiogram, and mechanocardiography.

In the present disclosure, the "biological sample" includes biologically derived cells, tissues, urine and blood samples, and the like. The biological sample is preferably the "blood sample".

The "blood sample" refers to blood (whole blood) collected from a test subject, or serum or plasma prepared from the blood. The blood sample is more preferably serum or plasma, and further preferably serum. The kind of the anticoagulant used when collecting plasma is not particularly limited. A blood sample of a test subject to be used in the measurement and a blood sample to determine a predetermined threshold may be the same or different kind, and are preferably the same kind. When plasma is used as the blood sample, plasma for determining a predetermined threshold is preferably prepared from a blood collected using the same anticoagulant as that used in the plasma of the test subject. The blood sample may be fresh or stored blood.

The biological sample is preferably collected within 2 hours after the test subject develops a symptom of heart disease and further preferably collected within 1 hour after the development. When serum or plasma is used as the biological sample, the biological sample is preferably prepared from a blood collected within 2 hours after the development and further preferably prepared from a blood collected within 1 hour after the development.

The biological sample may be subjected to the measurement immediately after collected or may be subjected to the measurement after frozen storage after collected.

When the blood sample is stored, frozen storage is possible, and it is also possible to separate plasma or serum immediately after collecting blood from the test subject, and freeze and store the separated plasma or serum.

The "peroxiredoxin" that is a marker of the present disclosure is an antioxidative enzyme having a peroxidase activity that reduces hydrogen peroxide to water, in the presence of a suitable electron donor.

Herein, it is sometimes called as "peroxiredoxin protein" when particularly referring to the protein of peroxiredoxin, and it is sometimes called as "peroxiredoxin gene" when particularly referring to the gene encoding peroxiredoxin.

The "measured value of peroxiredoxin" includes a "measured value of a peroxiredoxin protein" and an "expression level of a peroxiredoxin gene" in a biological sample.

The "measured value of a peroxiredoxin protein" refers to a value reflecting the amount or concentration of the peroxiredoxin protein. When the measured value is written by "amount", it may be written by mol or mass, and is preferably written by mass. When the value is written by "concentration", it may be written by mol concentration or a mass ratio per constant volume (mass/volume) of a biological sample, and is preferably written by mass/volume. As the value reflecting the amount or concentration, intensity of signal such as fluorescence and luminescence may be used, in addition to the those described above.

The "expression level of a peroxiredoxin gene" refers to a value reflecting an expression level of peroxiredoxin mRNA. The expression level may be represented by a copy number (absolute amount) of peroxiredoxin mRNA present in a constant amount of biological sample, or may be a value reflecting an expression level relative to the expression level of a house keeping gene such as β2-microglobulin mRNA, GAPDH mRNA and β-actin mRNA. The expression level may be expressed by intensity of signal such as fluorescence and luminescence.

The "predetermined threshold" refers to a threshold of the measured value of peroxiredoxin. The threshold can be determined based on the measured value of peroxiredoxin in a biological sample of an individual who has not developed acute myocardial infarction and/or the measured value of peroxiredoxin in a biological sample of an individual who has developed acute myocardial infarction.

For example, the measured values of peroxiredoxin measured using biological samples of a plurality of individuals who have developed acute myocardial infarction and the measured values of peroxiredoxin measured using biological samples of a plurality of individuals who have not developed acute myocardial infarction are obtained. The value capable of most accurately classifying into positive or negative based on a plurality of these values can be defined as a "threshold". The "value capable of most accurately classifying" can be appropriately set, based on indexes such as sensitivity, specificity, positive predictive value, and negative predictive value, according to the purpose of inspection.

For example, as one embodiment, among the measured values of peroxiredoxin in each biological sample obtained from a plurality of individuals who have developed acute myocardial infarction, the lowest measured value may be defined as a threshold. For example, when reducing false positive as much as possible, like a screening inspection, said threshold can be suitably used.

As another embodiment, when determining a threshold based on the measured values of peroxiredoxin in a biological sample of an individual who has not developed acute myocardial infarction, among the measured values of peroxiredoxin in biological samples obtained from a plurality of individuals who have not developed acute myocardial infarction, the highest measured value can be determined as a threshold. For example, when reducing false negative as much as possible, said threshold can be suitably used.

As another embodiment, a threshold can be also defined as the measured value of peroxiredoxin in a biological sample of an individual who has not developed acute myocardial infarction as it is, or an average, a median or a mode of a plurality of the measured values of peroxiredoxin of an individual who has not developed acute myocardial infarction.

A threshold can be also determined based on the measured values of peroxiredoxin in a plurality of biological samples of an individual who has not developed acute myocardial infarction.

In this case, a threshold can be defined as [the average of the measured values of peroxiredoxin] in a plurality of biological samples, preferably [a value obtained by subtracting "a value obtained by multiplying a standard deviation of the measured values of peroxiredoxin in a plurality of biological samples by one" from "the average"], or more preferably [a value obtained by subtracting "a value obtained by multiplying the standard deviation by two" from "the average"].

As a threshold, a past measured value (that may be one value, or may be an average, a median or a mode of a plurality of values) of peroxiredoxin obtained in the same test subject before the test subject has acute myocardial infarction can be also used.

When a threshold is determined based on the measured value of peroxiredoxin in a biological sample of an individual who has not developed acute myocardial infarction and the measured value of peroxiredoxin in a biological sample of an individual who has developed acute myocardial infarction, the average of the measured value of peroxiredoxin in a biological sample of a single individual who has not developed acute myocardial infarction and the measured value of peroxiredoxin in a biological sample of a single individual who has developed acute myocardial infarction can be used as a reference. A value obtained by further averaging "an average of the measured values of peroxiredoxin in a plurality of biological samples of an individual who has not developed acute myocardial infarction" and "an average of the measured values of peroxiredoxin in a plurality of biological samples of an individual who has developed acute myocardial infarction" can be also used as a threshold. As another embodiment, individuals who have not developed acute myocardial infarction and individuals who have developed acute myocardial infarction are defined as one group, and a median of the measured values of peroxiredoxin in biological samples of this one group can be also used as a threshold.

As still another embodiment, in the method of determining a threshold, in place of the measured value of peroxiredoxin in a biological sample of an individual who has not developed acute myocardial infarction, the measured value of peroxiredoxin in a biological sample of a healthy individual may be used.

These thresholds may be determined when obtaining the measured value of peroxiredoxin in a biological sample of a test subject, or may be previously determined.

The "healthy individual" is not particularly limited. Preferably, the "healthy individual" is a human or a mammal other than humans as described in the section of the "individual". The "healthy individual" refers to an individual that does not show abnormal data in a biochemical test, blood test, urine test, serum test, physiological test, and the like. Age and sex of the healthy individual are not particularly limited.

The "individual who has not developed acute myocardial infarction" is not particularly limited. Preferably, the "individual who has not developed acute myocardial infarction" is a human or a mammal other than humans as described in the section of the "individual". The "individual who has not developed acute myocardial infarction" is an individual who cannot be diagnosed to develop acute myocardial infarction, in accordance with known diagnosis criteria.

"A plurality of biological samples" is 2 or more, preferably 5 or more, and more preferably 10 or more biological samples. These may be biological samples collected from different individuals or may be a plurality of biological samples of the same individual collected at different timings.

"A plurality of values" is 2 or more, preferably 5 or more, and more preferably 10 or more measured values of peroxiredoxin.

"A plurality of individuals" is 2 or more, preferably 5 or more, and more preferably 10 or more individuals.

The species, age, sex and the like of the test subject are not necessarily the same as those of the individual to obtain the measured value of peroxiredoxin for determining a threshold, but they are preferably the same species. The individual is preferably the same generation and/or the same sex as the test subject.

In the present disclosure, peroxiredoxin to be measured is not limited as long as it is a peroxiredoxin family present in mammalian cells. The "measured value of a peroxiredoxin protein" is preferably a peroxiredoxin 1 group (PRDX1 variants 1 to 4), peroxiredoxin 5 (PRDX5) and peroxiredoxin 6 (PRDX6), and more preferably peroxiredoxin 6.

The "anti-peroxiredoxin antibody" is not limited as long as it specifically binds to a peroxiredoxin protein, and any of a polyclonal antibody, a monoclonal antibody and fragments thereof (for example, Fab, F(ab)₂, and the like) that are obtained by immunizing an animal other than humans with a peroxiredoxin protein or a part thereof as an antigen can be used. Class and subclass of immunoglobulin are not particularly limited. The class and subclass of immunoglobulin may be a chimeric antibody, scFv, or the like.

The peroxiredoxin protein to be an antigen used for preparing an anti-peroxiredoxin antibody is preferably a peroxiredoxin protein derived from human (for example, GenBank: NP_004896.1 [SEQ ID NO: 1]), mouse (for example, GenBank: AAP21829.1), rat (for example, NCBI Reference Sequence: NP_446028.1) or the like, and more preferably a human peroxiredoxin protein of SEQ ID NO: 1. The peroxiredoxin protein to be used as an antigen may be extracted from mammalian cells according to a known method, and may be a recombinant protein obtained according to a recombinant genetic engineering technique. When a part of the peroxiredoxin protein is used as an antigen, a fragment obtained by digesting the peroxiredoxin protein with an enzyme or the like may be used, and a peptide having the same sequence as the amino acid sequence of a part of the peroxiredoxin protein may be used as an antigen. The peptide can be synthesized by a known method.

As the anti-peroxiredoxin antibody, for example, a commercial product such as a peroxiredoxin 6 antibody (GTX115262; GeneTex) that reacts with a human peroxiredoxin protein can be also used.

In the present disclosure, the "peroxiredoxin mRNA" includes human (for example, NCBI Reference Sequence: NM_004905.2 [SEQ ID NO: 2]), mouse (for example, NCBI Reference Sequence: NM_007453.4, NM_001303408.1), rat (for example, NCBI Reference Sequence: NM_053576.2), and the like. The peroxiredoxin mRNA is more preferably human peroxiredoxin mRNA of SEQ ID NO: 2.

In the present disclosure, either total RNA or mRNA extracted from a biological sample may be used. The biological sample used in extraction of total RNA and mRNA is preferably subjected to RNA extraction shortly after being collected from an individual, or frozen (preferably rapidly cooled in an atmosphere of -80°C or less) shortly after being collected from an individual, and stored at -80°C or less until RNA extraction.

The method of extracting total RNA and mRNA from a biological sample is not particularly limited, and a known extraction method can be used.

### 2. Method for determining acute myocardial infarction

In the present embodiment, the measured value of peroxiredoxin in a biological sample is obtained, and whether or not a test subject has acute myocardial infarction is determined using the measured value of peroxiredoxin.

### (i) Step of obtaining measured value of peroxiredoxin

In the present step, first, a measured value of peroxiredoxin in a biological sample collected from a test subject is obtained.

A method for obtaining the measured value of peroxiredoxin in a biological sample is not particularly limited as long as the measured value of peroxiredoxin contained in the biological sample can be obtained.

### (i-1) Obtainment of measured value of peroxiredoxin protein

When the measured value of peroxiredoxin is a measured value of a peroxiredoxin protein, it is possible in the present step to use an antibody which is capable of specifically binding to the peroxiredoxin protein, namely, an anti-peroxiredoxin antibody, in order to obtain the measured value. The "4. An inspection reagent for acute myocardial infarction containing an anti-peroxiredoxin antibody" or "5. An inspection kit for acute myocardial infarction" described below may be used.

The order of mixing a biological sample and an anti-peroxiredoxin antibody is not particularly limited, and these may be mixed substantially at the same time, or may be successively mixed.

In the methods provided herein, a complex of an anti-peroxiredoxin antibody and a peroxiredoxin protein in a biological sample is formed in advance and the complex is then immobilized on a solid phase, or an anti-peroxiredoxin antibody is previously immobilized on a solid phase, and the immobilized anti-peroxiredoxin antibody and a peroxiredoxin protein in a biological sample can form a complex. More preferably, it is an embodiment that the complex is formed in advance and the complex is then immobilized on a solid phase. Moreover, the complex immobilized on a solid phase or the complex formed on a solid phase is detected by a method known in the art, whereby the amount or concentration of a peroxiredoxin protein contained in a biological sample can be measured.

When a complex of an anti-peroxiredoxin antibody and a peroxiredoxin protein in a biological sample is formed in advance and the complex is then immobilized on a solid phase, an anti-peroxiredoxin antibody modified with biotin or the like is brought into contact with a peroxiredoxin protein in a biological sample to form a complex. Separately, avidins are previously bound to the solid phase, whereby the complex can be immobilized on a solid phase via binding between biotin and avidins.

When an anti-peroxiredoxin antibody is previously immobilized on a solid phase, an embodiment of immobilization of an anti-peroxiredoxin antibody to a solid phase is not particularly limited. For example, an anti-peroxiredoxin antibody and a solid phase may be directly bound, or an anti-peroxiredoxin antibody and a solid phase may be indirectly bound via another substance. Examples of direct binding include physical adsorption and the like. Examples of indirect binding include binding via a combination of biotin with avidin or streptavidin (hereinafter, also referred to as "avidins"). In this case, an anti-peroxiredoxin antibody is previously modified with biotin, and avidins are previously bound to a solid phase, whereby an anti-peroxiredoxin antibody and a solid phase can be indirectly bound via the binding between biotin and avidins. In the present embodiment, the binding between an anti-peroxiredoxin antibody and a solid phase is preferably an indirect binding via biotin and avidins.

The material of the solid phase is not particularly limited. For example, the material of the solid phase can be selected from organic polymer compounds, inorganic compounds, biopolymers, and the like. Examples of the organic polymer compounds include latex, polystyrene, polypropylene, and the like. Examples of the inorganic compounds include magnetic bodies (iron oxide, chromium oxide and ferrite, and the like), silica, alumina, glass, and the like. Examples of the biopolymers include insoluble agarose, insoluble dextran, gelatin, cellulose, and the like. Two or more kinds of these materials may be used in combination. A shape of the solid phase is not particularly limited, and examples include a particle, a membrane, a microplate, a microtube, a test tube, and the like. Among them, a particle is preferred, and a magnetic particle is particularly preferred.

In the present step, after the formation of the complex, preferably after the complex formation and before detection of a labeling substance, B/F separation that removes unreacted free components not forming the complex may be carried out. The unreacted free component refers to a component that does not constituting a complex. Examples of the unreacted free component include an anti-peroxiredoxin antibody that does not bind to a peroxiredoxin protein and the like. Means of B/F separation is not particularly limited. When the solid phase is a particle, only a solid phase capturing the complex is collected by centrifugation, whereby B/F separation can be carried out. When the solid phase is a container such as a microplate or microtube, B/F separation can be carried out by removing a liquid containing unreacted free components. When the solid phase is a magnetic particle, B/F separation can be carried out by sucking and removing a liquid containing unreacted free components by a nozzle while magnetic particles are magnetically restricted with a magnet. The method is preferred from the viewpoint of automation. After removing unreacted free components, the solid phase capturing the complex may be washed with a suitable aqueous medium such as PBS.

In the present step, detection of the complex can be performed by using an anti-peroxiredoxin antibody labeled with a labeling substance, or can be performed by using an unlabeled anti-peroxiredoxin antibody and an anti-immunoglobulin antibody or the like labeled with a labeling substance capable of binding to the unlabeled anti-peroxiredoxin antibody, and it is preferred to use a labeled anti-peroxiredoxin antibody. An epitope in a peroxiredoxin protein of the labeled anti-peroxiredoxin antibody and an epitope in a peroxiredoxin protein of the anti-peroxiredoxin antibody bound to the solid phase are preferably different.

The labeling substance used for a labeled anti-peroxiredoxin antibody or labeled anti-immunoglobulin antibody is not particularly limited, as long as a detectable signal is generated. For example, the labeling substance may be a substance generating a signal in itself (hereinafter, also referred to as "signal-generating substance"), or may be a substance generating a signal by catalyzing a reaction of other substance. Examples of the signal-generating substance include fluorescent substances, radioisotopes, and the like. Examples of the substance generating a signal detectable by catalyzing a reaction of other substance include enzymes and the like. Examples of the enzymes include alkaline phosphatase, peroxidase, β-galactosidase, luciferase, and the like. Examples of the fluorescent substances include fluorescent dyes such as fluorescein isothiocyanate (FITC), rhodamine and Alexa Fluor (registered trademark), fluorescent proteins such as GFP, and the like. Examples of the radioisotopes include ¹²⁵I, ¹⁴C, ³²P, and the like. Among them, as the labeling substance, an enzyme is preferred, and alkaline phosphatase is particularly preferred.

The labeled anti-peroxiredoxin antibody is obtained by labeling an anti-peroxiredoxin antibody with the above labeling substance, according to a labeling method known in the art. An anti-peroxiredoxin antibody may be labeled using a commercially available labeling kit or the like. The labeled immunoglobulin antibody may be obtained by using the same method as labeling of an anti-peroxiredoxin antibody, or a commercially available product may be used.

In the present step, a signal generated by the labeling substance of the labeled anti-peroxiredoxin antibody contained in the complex is detected, whereby a measured value of peroxiredoxin contained in the biological sample can be obtained. The phrase "a signal is detected" includes qualitatively detecting the presence or absence of a signal, quantifying signal intensity, and semiquantitatively detecting signal intensity. The semiquantitative detection refers to indication of signal intensity in stages like "no signal generated", "weak", "middle", or "strong". In the present step, it is preferred that signal intensity is detected quantitatively or semiquantitatively.

The method itself for detecting a signal is known in the art. In the present step, a measurement method according to the kind of the signal derived from the above labeling substance may be properly selected. For example, when the labeling substance is an enzyme, a signal of light or color generated by reacting a substrate on the enzyme can be measured using a known device such as a luminometer or spectrophotometer.

The substrate of enzyme can be properly selected from known substrates according to the kind of the enzyme. When alkaline phosphatase is used as the enzyme, examples of the substrate include chemiluminescent substrates such as CDP-Star (registered trademark) (disodium 4-chloro-3-(methoxyspiro[1,2-dioxetane-3,2'-(5'-chloro)tricyclo[3.3.1.13,7]decan]-4-yl)phenyl phosphate) and CSPD (registered trademark) (disodium 3-(4-methoxyspiro[1,2-dioxetane-3,2-(5'-chloro)tricyclo[3.3.1.13,7]decan]-4-yl)phenyl phosphate), and chromogenic substrates such as 5-bromo-4-chloro-3-indolyl phosphate (BCIP), disodium 5-bromo-6-chloro-indolyl phosphate and p-nitrophenyl phosphoric acid. Particularly preferred is CDP-Star (registered trademark). Luminescence of the substrate is preferably detected by a luminometer.

When the labeling substance is a radioisotope, radiation as a signal can be measured using a known device such as a scintillation counter. When the labeling substance is a fluorescent substance, fluorescence as a signal can be measured using a known device such as a fluorescence microplate leader. Excitation wavelength and fluorescence wavelength can be properly determined according to the kind of the used fluorescent substance.

The detection result of signal can be used as the measured value of a peroxiredoxin protein. For example, when the signal intensity is quantitatively detected, the measured value of signal intensity itself or the value calculated from the measured value of signal intensity can be used as the measured value of a peroxiredoxin protein. Examples of the value calculated from the measured value of signal intensity include a value obtained by subtracting a measured value of signal intensity of a negative control sample from the measured value of signal intensity, a value obtained by dividing the measured value of signal intensity by a measured value of signal intensity of a positive control sample, a combination thereof, and the like. Examples of the negative control sample include a biological sample of a healthy subject, and the like. Examples of the positive control sample include a biological sample containing a peroxiredoxin protein in a predetermined amount or concentration.

In order to obtain the measured value of a peroxiredoxin protein, for example, a commercially available ELISA kit detecting a human peroxiredoxin protein such as ELISA Kit for Peroxiredoxin 6 (Cat. No. MBS2020865) of MyBioSource, Inc. (San Diego, California, USA) can be also used. When the measured value of peroxiredoxin in a biological sample is obtained using a commercially available kit, the measured value can be obtained according to the protocol attached to the kit.

### (i-2) Obtainment of expression level of peroxiredoxin gene

When the measured value of peroxiredoxin is an expression level of a peroxiredoxin gene, a known method such as a microarray method, an RNA-seq analysis method or a quantitative RT-PCR method can be used for obtaining the expression level in the present step. As for a probe used in the microarray method, a probe selected by itself or a known probe may be synthesized and used, or a commercially available microarray chip may be used. Quantification by the microarray method can be carried out according to a known method, and the expression level of peroxiredoxin mRNA may be expressed as an expression level relative to the expression level of housekeeping gene, and can be expressed as a measured value of signal intensity of a fluorescent dye or the like.

Quantification by RT-PCR can be carried out by performing a reverse transcription reaction using total RNA or mRNA extracted from a biological sample as a template, and analyzing by a realtime PCR method or the like using a specific primer of peroxiredoxin mRNA using the obtained cDNA as a template. In this case, the expression level of peroxiredoxin mRNA may be expressed as an expression level relative to the expression level of housekeeping gene, and may be expressed as a measured value of intensity of signal such as a fluorescent dye.

In the RNA-seq analysis method, first, the mRNA extracted from a biological sample is fragmented, and synthesis and library creation of cDNA are performed by a reverse transcription reaction using the fragmented mRNA as a template. As to the fragment contained in each library, a base sequence is determined by a next generation sequencer, and the information is mapped to a reference gene sequence, then the expression level of mRNA is expressed as RPKM (Reads Per Killobases per Million). RPKM may be expressed as intensity of signal such as heat map.

The detection result of the signal can be used as the expression level of peroxiredoxin mRNA. For example, when the signal intensity is quantitatively detected, the measured value of signal intensity itself or the value calculated from the measured value of signal intensity can be used as the expression level of peroxiredoxin mRNA.

Examples of the value calculated from the measured value of signal intensity include a value obtained by subtracting a measured value of signal intensity of a negative control sample from the measured value of signal intensity, a value obtained by dividing the measured value of signal intensity by a measured value of signal intensity of a positive control sample, a combination thereof, and the like. Examples of the negative control sample include a biological sample of a healthy subject, and the like. Examples of the positive control sample include a biological sample containing peroxiredoxin mRNA in a predetermined expression level.

### (ii) Step of obtaining information on acute myocardial infarction based on measured value of peroxiredoxin

Next, information on acute myocardial infarction is obtained based on the measured value of peroxiredoxin obtained in the step (i) above. More specifically, the measured value of peroxiredoxin in a biological sample of a test subject and the predetermined threshold are compared according to a known method such as a simple comparison method and a statistical test. Subsequently, when the measured value of peroxiredoxin in a biological sample of a test subject is the threshold or more, it can be determined that the test subject has acute myocardial infarction or is suspected to have acute myocardial infarction. When the measured value of peroxiredoxin in a biological sample of a test subject is lower than the threshold, it can be determined that the test subject does not have acute myocardial infarction. The information on acute myocardial infarction is, for example, information on whether or not the test subject has acute myocardial infarction, information on whether or not the test subject is suspected to have acute myocardial infarction, and the like, obtained by the present step. Preferably, when the measured value of peroxiredoxin in a biological sample of a test subject is the threshold or more, information showing that a test subject has acute myocardial infarction is obtained. The obtained information is provided for supporting a doctor to diagnose that the test subject has acute myocardial infarction or to diagnose that the test subject does not have acute myocardial infarction.

The description concerning "test subject", "biological sample", "measured value of peroxiredoxin", "predetermined threshold" and the like described in the "1. Explanation of terms" above may be incorporated herein.

### 3. Biomarker for determining acute myocardial infarction

The present disclosure includes a biomarker for determining whether or not a test subject has acute myocardial infarction, in the "2. Method for determining acute myocardial infarction" described above. The biomarker contains a peroxiredoxin protein present in a biological sample of an individual, preferably a peptide containing all or part of the amino acid sequence of SEQ ID NO: 1 or 2, and peroxiredoxin mRNA, preferably a nucleic acid containing all or part of the base sequence of SEQ ID NO: 3 or 4. In the present embodiment, the biomarker in a biological sample of a test subject is measured, and whether or not the test subject has acute myocardial infarction can be determined based on the obtained measured value.

The description concerning "test subject", "biological sample", "measured value of peroxiredoxin", "predetermined threshold" and the like described in the "1. Explanation of terms" above may be incorporated herein.

### 4. Inspection reagent for acute myocardial infarction containing anti-peroxiredoxin antibody

The present disclosure provides an inspection reagent for acute myocardial infarction containing an anti-peroxiredoxin antibody used in the "2. Method for determining acute myocardial infarction" described above.

As the anti-peroxiredoxin antibody, those described in the "1. Explanation of terms" described above can be used.

The inspection reagent of the present embodiment may contain at least one or more anti-peroxiredoxin antibodies. When the anti-peroxiredoxin antibody is a polyclonal antibody, the anti-peroxiredoxin antibody may be a polyclonal antibody obtained by immunization with one kind of antigen, or the anti-peroxiredoxin antibody may be a polyclonal antibody obtained by immunizing the same individual with two or more kinds of antigens in parallel. Polyclonal antibodies each obtained by respectively inoculating two or more kinds of antigens into different animals may be mixed. When the anti-peroxiredoxin antibody is a monoclonal antibody, the anti-peroxiredoxin antibody may be a monoclonal antibody produced from one kind of hybridoma, or the anti-peroxiredoxin antibody may be a monoclonal antibody produced from two or more kinds of hybridomas, and two or more kinds of a plurality of monoclonal antibodies in which each monoclonal antibody recognizes the same or different epitope may be contained. One or more kinds of polyclonal antibodies and one or more kinds of monoclonal antibodies may be mixed and contained.

The form of an anti-peroxiredoxin antibody contained in the inspection reagent is not particularly limited. The form of an anti-peroxiredoxin antibody contained in the inspection reagent may be a dry state or liquid state of antisera or ascites containing an anti-peroxiredoxin antibody or the like. The form of an anti-peroxiredoxin antibody may be a dry state or an aqueous solution of a purified anti-peroxiredoxin antibody, an immunoglobulin fraction containing an anti-peroxiredoxin antibody or an IgG fraction containing an anti-peroxiredoxin antibody.

When the form is a dry state or liquid state of antisera or ascites containing an anti-peroxiredoxin antibody, at least one of stabilizers such as β-melcaptoethanol and DTT; protective agents such as albumin; surfactants such as polyoxyethylene(20) sorbitan monolaurate and polyoxyethylene(10) octylphenyl ether; preservatives such as sodium azide and the like may be contained. When the form of an anti-peroxiredoxin antibody is a dry state or an aqueous solution of a purified anti-peroxiredoxin antibody, an immunoglobulin fraction containing an anti-peroxiredoxin antibody or an IgG fraction containing an anti-peroxiredoxin antibody, at least one of buffer components such as a phosphate buffer solution; stabilizers such as β-melcaptoethanol and DTT; protective agents such as albumin; salts such as sodium chloride; surfactants such as polyoxyethylene(20) sorbitan monolaurate and polyoxyethylene(10) octylphenyl ether; and preservatives such as sodium azide may be contained.

In the present disclosure, the anti-peroxiredoxin antibody may be unlabeled, or may be labeled with biotin or the above-described labeling substance. The anti-peroxiredoxin antibody is preferably labeled with biotin or the above-described labeling substance. As the labeling substance, those exemplified in the section of the "2. Method for determining acute myocardial infarction" described above can be used, and alkaline phosphatase is preferably used. In the present disclosure, an anti-peroxiredoxin antibody may be provided in a state immobilized on the solid phase surface or the like. The solid phase and immobilization are as exemplified in the section of the "2. Method for determining acute myocardial infarction" described above. The solid phase is preferably a magnetic bead.

### 5. Inspection kit for acute myocardial infarction

The present disclosure is an inspection kit for acute myocardial infarction containing the "4. An inspection reagent for acute myocardial infarction containing an anti-peroxiredoxin antibody" described above, used in a method for detecting an acute myocardial infarction patient or a method for identifying heart disease.

More specifically, the present disclosure is an inspection kit for acute myocardial infarction containing an inspection reagent containing an anti-peroxiredoxin antibody labeled with biotin and an inspection reagent containing an anti-peroxiredoxin antibody labeled with a labeled substance. The labeling substance is preferably alkaline phosphatase. The kit may further contain a solid phase, preferably a solid phase to which avidins are bound, and more preferably a magnetic bead to which avidins are bound. The kit may contain a substrate. The substrate is preferably CDP-Star (registered trademark).

Details of the labeling substance, solid phase and substrate are as described in the "2. Method for determining acute myocardial infarction", and the description may be incorporated herein.

The inspection kit preferably contains two kinds of anti-peroxiredoxin antibodies (first anti-peroxiredoxin antibody, second anti-peroxiredoxin antibody) bound to different epitopes on peroxiredoxin proteins. In this case, a complex of a first anti-peroxiredoxin antibody, peroxiredoxin, a second anti-peroxiredoxin antibody and a labeling substance is formed on a solid phase. The detection method as described above is generally called as sandwich ELISA. In this complex, the first anti-peroxiredoxin antibody is immobilized on the solid phase, and the second anti-peroxiredoxin antibody is directly or indirectly bound to the labeling substance. The phrase "the second anti-peroxiredoxin antibody and the labeling substance are indirectly bound" refers to that the labeling substance is bound to the second anti-peroxiredoxin antibody via an antibody or the like. Examples include a state that the labeling antibody recognizing the second anti-peroxiredoxin antibody is bound to the second anti-peroxiredoxin antibody.

When the first anti-peroxiredoxin antibody is previously bound to the solid phase, the inspection kit can contain the solid phase on which the first anti-peroxiredoxin antibody is immobilized, the second anti-peroxiredoxin antibody, and the labeling substance. The second anti-peroxiredoxin antibody and the labeling substance may be stored in separate containers, or may be stored in the same container. When the labeling substance is an enzyme and the inspection kit further contains a substrate, the enzyme and the substrate need to be stored in separate containers. When provided to a user, at least two kinds among the solid phase, the second anti-peroxiredoxin antibody and the labeling substance may be packed together, or may be separately packed.

When the first anti-peroxiredoxin antibody is not previously bound to the solid phase, the inspection kit can contain the solid phase, the first anti-peroxiredoxin antibody, the second anti-peroxiredoxin antibody, and the labeling substance. At least two kinds among the first anti-peroxiredoxin antibody, the second anti-peroxiredoxin antibody and the labeling substance may be stored in the same container, or may be stored in separate containers. When the labeling substance is an enzyme and the inspection kit further contains a substrate, the enzyme and the substrate need to be stored in separate containers. When provided to a user, at least two kinds among the solid phase, the first anti-peroxiredoxin antibody, the second anti-peroxiredoxin antibody and the labeling substance may be packed together, or may be separately packed.

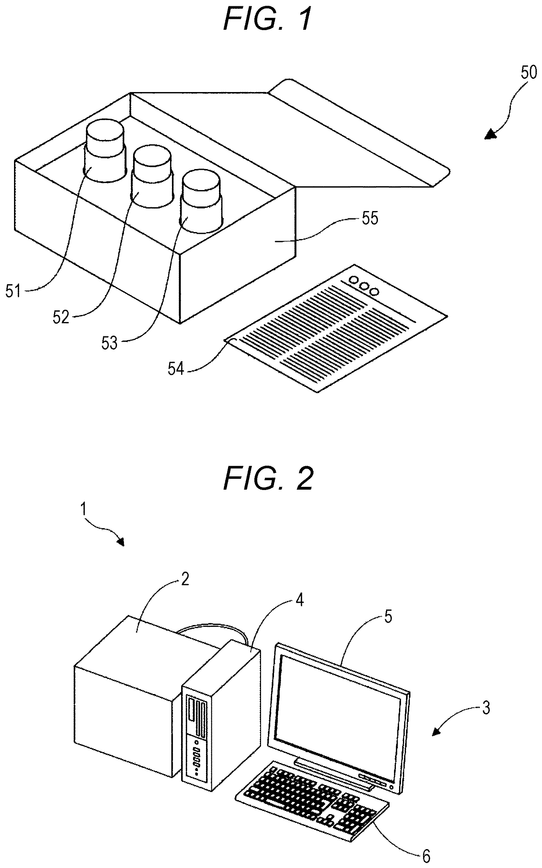

The above-described inspection kit can be provided to a user as a kit as shown in Fig. 1. An inspection kit 50 for acute myocardial infarction contains an outer packaging box 55, a first container 51 containing a plurality of magnetic particles as a solid phase, a second container 52 containing a first anti-peroxiredoxin antibody which can be bound to the solid phase, a third container 53 containing an enzyme-labelled second anti-peroxiredoxin antibody, and an attached document 54 of the inspection kit. In the attached document 54, a method for handling an inspection kit, storage conditions, an expiration date, and the like can be described. A container containing a substrate reagent, a container containing an aqueous medium for washing and the like may be packed together in the packaging box 55.

The present application also provides the use of an anti-peroxiredoxin antibody, for producing an inspection reagent or inspection kit for acute myocardial infarction. The inspection reagent, inspection kit and anti-peroxiredoxin antibody are as described above.

### 6. Device for supporting diagnosis of acute myocardial infarction, and program for supporting diagnosis of acute myocardial infarction

Hereinbelow, an embodiment of a diagnosis supporting device and diagnosis supporting program for performing the methods described above will be described with reference to the attached drawings. Fig. 2 is a schematic view of a diagnosis supporting device 1. The diagnosis supporting device 1 contains a measurement device 2 and a computer system 3 connected to the measurement device 2.

The measurement device 2 measures a measured value of peroxiredoxin in a biological sample collected from a test subject. The measurement device 2 is not particularly limited. The measurement device 2 can be properly selected depending on the method for measuring peroxiredoxin. When the measured value of peroxiredoxin is a measured value of a peroxiredoxin protein, the measurement device 2 is a measurement device capable of detecting a signal generated by ELISA method using a biotin-labeled anti-peroxiredoxin antibody, magnetic particles on which avidins are immobilized, and an anti-peroxiredoxin antibody labeled with a labeling substrate. This kind of measurement device is not particularly limited, as long as it is possible to detect a signal based on the used labeling substrate. The measurement device can be properly selected according to the kind of the labeling substrate.

When a biotin-labeled anti-peroxiredoxin antibody, an inspection reagent containing magnetic particles on which avidins are immobilized, and a reagent containing an anti-peroxiredoxin antibody labeled with a labeling substrate and a biological sample collected from a test subject are set in the measurement device 2, the measurement device 2 performs antigen-antibody reaction using each reagent. The measurement device 2 obtains a signal as an optical information based on a labeled antibody specifically bound to peroxiredoxin. The measurement device 2 sends the obtained optical information to the computer system 3.

When the measured value of peroxiredoxin is an expression level of a peroxiredoxin gene, the measurement device 2 is a measurement device capable of detecting a signal generated by any of a microarray analysis, an RNA-seq analysis or a quantitative RT-PCR analysis. This kind of measurement device is not particularly limited, as long as it is possible to detect a signal based on the used labeling substrate. The measurement device can be properly selected according to the kind of the labeling substrate.

When mRNA extracted from a biological sample collected from a test subject or a reverse transcription product of the mRNA (may be labeled with a fluorescent dye or the like as necessary) is set in the measurement device 2 having a function to perform any processing of a microarray analysis, an RNA-seq analysis and a quantitative RT-PCR analysis, the measurement device 2 performs a predetermined processing. The measurement device 2 obtains a signal as an optical information based on the expression level of the peroxiredoxin mRNA. The measurement device 2 sends the obtained optical information to the computer system 3.

The computer system 3 contains a computer 4, an input part 5 for inputting data and the like, and a display part 6 for displaying information of a test subject, a detection result and the like. The computer system 3 obtains a measured value of peroxiredoxin in a biological sample collected from a test subject, based on the optical information received from the measurement device 2. The computer system 3 detects whether or not the test subject has acute myocardial infarction, based on the measured value of peroxiredoxin. The computer system 3 may be a separate equipment from the measurement device 2, as shown in Fig. 2, or the computer system 3 may be incorporated in the measurement device 2.

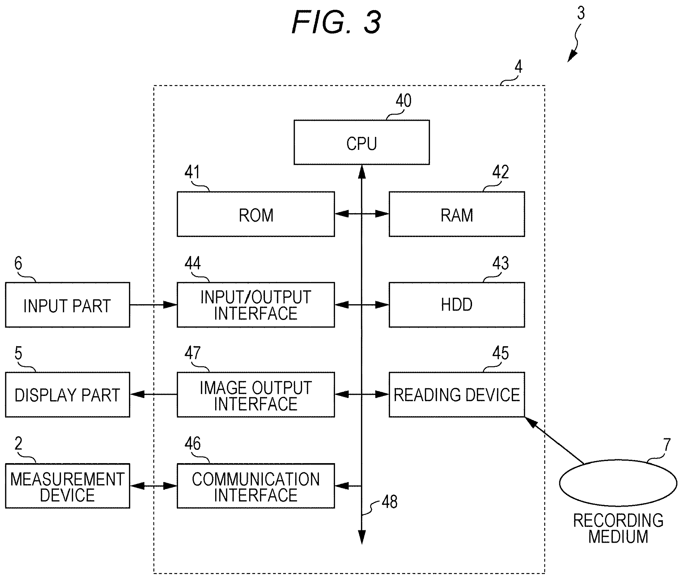

The computer 4 includes a processor (CPU) 40, an ROM 41, an RAM 42, a hard disk (HDD) 43, an input/output interface 44, a reading device 45, a communication interface 46 and an image output interface 47, as shown in Fig. 3, and these are connected via a bus 48 in a data-communicable manner. The measurement device 2 is communicably connected with the computer 4 by the communication interface 46.

The CPU 40 serially controls operation of each input and output part. The CPU 40 also executes a computer program stored in the ROM 41 or the hard disk 43. Namely, the CPU 40 processes the optical information received from the measurement device 2, in accordance with the computer program. The CPU 40 calculates the measured value of peroxiredoxin in a biological sample. The CPU 40 also reads a predetermined threshold (cut off value) stored in the ROM 41 or the hard disk 43, and when the measured value of peroxiredoxin is the predetermined threshold or more, the test subject collected from a biological sample is determined to have acute myocardial infarction. Moreover, the CPU 40 outputs a determination result to display it on the display part 6. Accordingly, the application also provides a computer program comprising instructions which, when the program is executed by a computer, cause the computer to carry out the steps of the method of supporting a diagnosis of acute myocardial infarction.

The ROM 41 comprises a mask ROM, a PROM, an EPROM, an EEPROM, and the like. In the ROM 41, a computer program for detection of acute myocardial infarction (program for supporting a diagnosis of acute myocardial infarction) to be executed by the CPU 40 and a data used for executing the program for supporting a diagnosis of acute myocardial infarction are recorded, as described above. In the ROM 41, in addition to the predetermined threshold, the past measured value of peroxiredoxin of the test subject and the like may be recorded.

The RAM 42 comprises a SRAM, a DRAM, and the like. The RAM 42 is used for reading computer programs recorded in the ROM 41 and the hard disk 43. The RAM 42 is used as a working area of the CPU 40 when the CPU 40 executes these computer programs.

In the hard disk 43, an operation system and computer programs such as an application program (program for supporting a diagnosis of acute myocardial infarction) for making the CPU 40 execute and a data used for executing the computer programs are recorded. In the hard disk 43, in addition to the predetermined threshold (cut off value), the past measured value of peroxiredoxin of the test subject and the like may be recorded.

The reading device 45 comprises a flexible disk drive, a CD-ROM drive, a DVD-ROM drive, and the like. The reading device 45 can read a computer program or data recorded in a portable recording medium 7.

The input/output interface 44 comprises, for example, a serial interface such as USB, IEEE1394 or RS-232C, a parallel interface such as SCSI, IDE or IEEE1284, and an analogue interface comprising a D/A converter, an A/D converter or the like. To the input/output interface 44, the input part 5 such as a keyboard and a mouse is connected. An operator can input various commands to the computer 4 by the input part 5.

Examples of the communication interface 46 are Ethernet (registered trademark) interface and the like. The computer 4 can send print data to a printer or the like, through the communication interface 46.

The image output interface 47 is connected to the display part 6 comprising LCD, CRT and the like. The display part 6 can output a video signal corresponding to the image data given from the CPU 40. The display part 6 displays an image according to an input video signal.

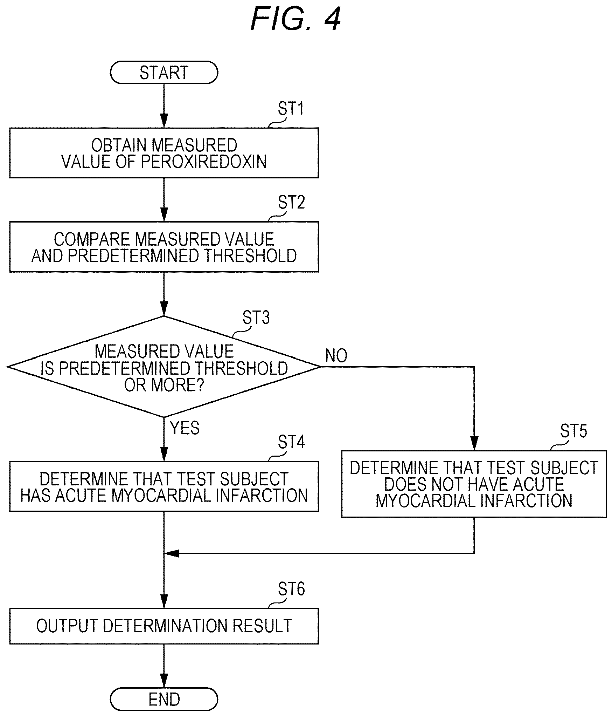

Next, a method for supporting a diagnosis of acute myocardial infarction executed by the diagnosis supporting device 1 will be described, based on the program for supporting a diagnosis of acute myocardial infarction, using Fig. 4. First, in Step ST1, when the CPU 40 receives transmission of optical information (signal) from the measurement device 2, the CPU 40 calculates the measured value of peroxiredoxin in a biological sample from the obtained optical information. The CPU 40 stores the calculated measured value in the ROM 41 or the hard disk 43. Then, in Step ST2, the CPU 40 compares the obtained measured value of peroxiredoxin and the predetermined threshold (cut off value) stored in the ROM 41 or the hard disk 43. When the measured value of peroxiredoxin is the predetermined threshold (cut off value) or more, it is "YES" in Step ST3 and proceeds to ST4, then the CPU 40 determines that the test subject from which the biological sample is collected has acute myocardial infarction, and in Step ST6, the CPU 40 outputs a determination result to display it on the display part 6, and print it out by a printer. The result that the test subject from which the biological sample is collected has acute myocardial infarction may be stored in the ROM 41 or the hard disk 43.

On the other hand, when the measured value of peroxiredoxin is lower than the predetermined threshold (cut off value), it is "NO" in Step ST3 and proceeds to ST5, and the CPU 40 determines that the test subject from which the biological sample is collected does not have acute myocardial infarction. Then, in Step ST6, the CPU 40 outputs a determination result to display it on the display part 6, and print it out by a printer. The result that the test subject from which the biological sample is collected does not have acute myocardial infarction may be stored in the ROM 41 or the hard disk 43.

Such information is provided to a doctor, as information for supporting to diagnose that the test subject has acute myocardial infarction or to diagnose that the test subject does not have acute myocardial infarction.

An embodiment of a device for supporting a diagnosis of acute myocardial infarction and a program for supporting a diagnosis of acute myocardial infarction according to the present disclosure was described as above. However, the present disclosure is not limited to the above embodiment, and various modifications can be made without deviating from the gist of the present disclosure.

### EXAMPLES

Hereinbelow, the present invention will be further described in detail, with reference to Examples, but the present invention is not construed as being limited to the embodiments of the Examples.

### Examples: Correlation between measurement of peroxiredoxin concentration in serum and various heart diseases

In order to compare the peroxiredoxin concentration in the serum of each of heart disease patients, the peroxiredoxin concentration in the serum of each test subject in five groups shown in Table 1 was measured.

### 1. Subjects

The details of test subjects are shown in Table 1.

**[Table 1]**

| Test subject | Number of people |
|---|---|
| Acute myocardial infarction (AMI) patient group | 10 |
| Ischemic heart failure (IHF) patient group | 15 |
| Atrial fibrillation (Af) patient group | 10 |
| Dilated cardiomyopathy (DCM) patient group | 15 |
| Healthy subject group | 10 |

A healthy subject is a person who is not suspected of a disease including not only a heart disease, but also other diseases.

### 2. Measurement method

Blood was collected from a test subject, and the serum was separated immediately after blood collection. Then, the separated serum was immediately frozen at -80°C. The frozen serum was stored at -80°C until measurement.

Peroxiredoxin in the serum was measured according to the attached protocol, using ELISA Kit for Peroxiredoxin 6 (Cat. No. MBS2020865) purchased from MyBioSource, Inc. (San Diego, California, USA).

StatFlex was used for statistical analysis, and fundamental statistic (parametric method), one-way analysis of variance and independent multigroup multiple comparison (Tukey's test) were performed. ROC analysis was performed for tests of sensitivity and specificity, and the AUC value was further obtained.

### 3. Result (1) Peroxiredoxin 6 concentration in serum

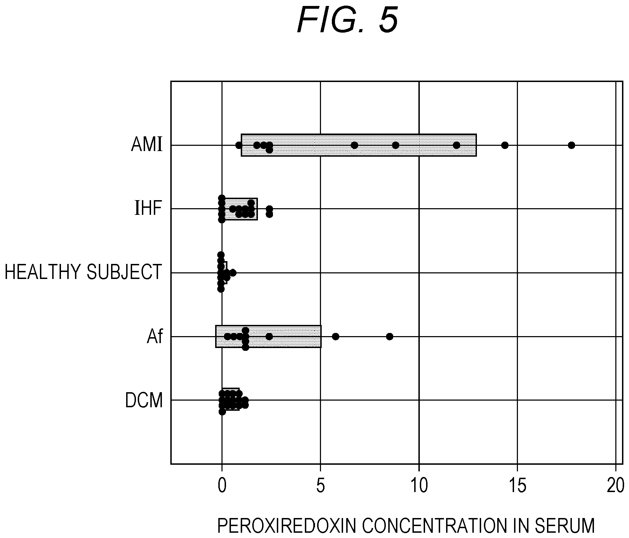

Average ± standard deviation (ng/ml) of the peroxiredoxin 6 concentration in the serum of each group obtained by a parametric method is shown in Table 2. A scatter diagram of a peroxiredoxin concentration in the serum of each group is shown in Fig. 5.

**[Table 2]**

| | Peroxiredoxin 6 concentration in serum (ng/ml) |
|---|---|
| AMI patient group | 6.94 ± 6.00 |
| IHF patient group | 0.95 ± 0.85 |
| Af patient group | 2.35 ± 2.69 |
| DCM patient group | 0.49 ± 0.42 |
| Healthy subject group | 0.10 ± 0.17 |

The results of Tukey's test are shown in Table 3. As shown in Table 3, q values of the AMI patient group against other groups all showed P<0.01, thus it was shown that the measured value of peroxiredoxin in the serum was significantly increased in the AMI patient group as compared to other disease groups.

**[Table 3]**

| | | AMI patient group | IHF patient group | Af patient group | DCM patient group | Healthy subject group |
|---|---|---|---|---|---|---|
| AMI patient group | q value Significance | | -8.1945 | -5.7356 | -8.8293 | -8.5894 |
| | | | P < 0.01 | P < 0.01 | P < 0.01 | P < 0.01 |
| IHF patient group | q value Significance | 8.1945 | | 1.9115 | -0.7097 | -1.1600 |
| | | P < 0.01 | | NS | NS | NS |
| Af patient group | q value Significance | 5.7356 | -1.9115 | | -2.5463 | -2.803 |
| | | P < 0.01 | NS | | NS | NS |
| DCM patient group | q value Significance | 8.8293 | 0.7097 | 2.5463 | | -0.5252 |
| | | P < 0.01 | NS | NS | | NS |
| Healthy subject group | q value Significance | 8.5394 | 1.1600 | 2.803 | 0.5252 | |
| | | P < 0.01 | NS | NS | NS | |

Next, ROC analysis was performed to study the sensitivity and specificity of the measured value of peroxiredoxin 6 in the prediction or diagnosis of acute myocardial infarction. ROC curves of the measured value of peroxiredoxin 6 of the AMI patient group for the measured values of peroxiredoxin 6 of the healthy subject group and each of the heart disease patient groups are shown in Fig. 6. The ROC curves of the AMI patient group had a shape along the upper left corner. AUC values for the AMI patient group against each of the disease groups and AUC values of the healthy subject group against each of the disease groups are shown in Table 4. The AUC values for the AMI patient group against the IHF patient group or the DMC patient group were 0.92 and 0.99 that were classified into high accuracy (Table 4). On the other hand, the AUC values for the healthy subject group against the IHF patient group or the DMC patient group were 0.79 and 0.82 that were classified into low accuracy (Table 4). Based on the above, it became clear that the measured value of peroxiredoxin is excellent in predictive ability or diagnostic ability of acute myocardial infarction. The measured value of peroxiredoxin increases within one hour after a symptom of heart disease associated with myocardial infarction appears. Therefore, it was considered that peroxiredoxin is useful in the detection of acute myocardial infarction immediately after onset. It was considered that the above detection can detect acute myocardial infarction earlier than the measured values of cardiac troponin T and cardiac troponin I conventionally reported.

**[Table 4]**

| | AMI patient group | Healthy subject group |
|---|---|---|
| Against IHF patient group | 0.92 | 0.79 |
| Against Af patient group | 0.81 | 0.99 |
| Against DCM patient group | 0.99 | 0.82 |
| Against healthy subject group | 1.00 | |

## Claims

1. A method for supporting a diagnosis of acute myocardial infarction comprising:
(a) a step of obtaining a measured value of peroxiredoxin in a biological sample collected from a test subject, and
(b) a step of obtaining information on acute myocardial infarction, comprising comparing the measured value of peroxiredoxin and a predetermined threshold,
wherein the information showing that the test subject has acute myocardial infarction or is suspected to have acute myocardial infarction is obtained when the measured value is the threshold or more.

2. The method according to claim 1, wherein the biological sample is a biological sample collected within 2 hours after the test subject develops a symptom of heart disease.

3. The method according to claim 1 or 2, wherein the biological sample is a blood sample.

4. The method according to any one of claims 1 to 3, wherein, in the step (b), the information showing that the test subject does not have acute myocardial infarction is obtained when the measured value is less than the threshold.

5. The method according to any one of claims 1 to 4, wherein the measured value of peroxiredoxin is an expression level of a peroxiredoxin gene or a measured value of a peroxiredoxin protein.

6. Use of a reagent for the method for supporting a diagnosis of acute myocardial infarction according to any one of claims 1 to 5, wherein
the reagent comprises an anti-peroxiredoxin antibody.

7. Use of a kit for the method for supporting a diagnosis of acute myocardial infarction according to any one of claims 1 to 5, the kit comprising:
a first anti-peroxiredoxin antibody;
a solid phase;
a second anti-peroxiredoxin antibody and a labeling substance.

## Patentansprüche

1. Verfahren zum Unterstützen einer Diagnose eines akuten Myokardinfarkts, umfassend:
(a) einen Schritt des Erhaltens eines Messwertes von Peroxiredoxin in einer biologischen Probe, die von einer Testperson gesammelt wurde, und
(b) einen Schritt des Erhaltens von Information über akuten Myokardinfarkt, umfassend das Vergleichen des Messwerts von Peroxiredoxin und einer festgelegten Schwelle, wobei die Information, die zeigt, dass die Testperson einen akuten Myokardinfarkt hat oder unter dem Verdacht steht, einen akuten Myokardinfarkt zu haben, erhalten wird, wenn der Messwert gleich der Schwelle ist oder darüber liegt.

2. Verfahren nach Anspruch 1, wobei die biologische Probe eine biologische Probe ist, die innerhalb von 2 Stunden gesammelt wird, nachdem die Testperson ein Symptom einer Herzerkrankung entwickelt hat.

3. Verfahren nach Anspruch 1 oder 2, wobei die biologische Probe eine Blutprobe ist.

4. Verfahren nach einem der Ansprüche 1 bis 3, wobei in Schritt (b) die Information, die zeigt, dass die Testperson keinen akuten Myokardinfarkt hat, erhalten wird, wenn der Messwert unter der Schwelle liegt.

5. Verfahren nach einem der Ansprüche 1 bis 4, wobei der Messwert von Peroxiredoxin ein Expressionsniveau eines Peroxiredoxingens oder ein Messwert eines Peroxiredoxinproteins ist.

6. Verwendung eines Reagens für das Verfahren zur Unterstützung einer Diagnose eines akuten Myokardinfarkts nach einem der Ansprüche 1 bis 5, wobei
das Reagens einen anti-Peroxiredoxin-Antikörper umfasst.

7. Verwendung eines Kits für das Verfahren zur Unterstützung einer Diagnose eines akuten Myokardinfarkts nach einem der Ansprüche 1 bis 5, wobei das Kit umfasst:
einen ersten anti-Peroxiredoxin-Antikörper;
eine feste Phase;
einen zweiten anti-Peroxiredoxin-Antikörper und eine Markierungssubstanz.

## Revendications

1. Procédé pour soutenir un diagnostic d'infarctus du myocarde aigu comprenant :
(a) une étape d'obtention d'une valeur mesurée de peroxyrédoxine dans un échantillon biologique collecté à partir d'un sujet d'essai, et
(b) une étape d'obtention d'informations sur un infarctus du myocarde aigu, comprenant la comparaison de la valeur mesurée de peroxyrédoxine et d'un seuil prédéterminé, dans lequel les informations montrant que le sujet d'essai a un infarctus du myocarde aigu ou est suspecté d'avoir un infarctus du myocarde aigu sont obtenues lorsque la valeur mesurée est le seuil ou plus.

2. Procédé selon la revendication 1, dans lequel l'échantillon biologique est un échantillon biologique collecté au plus 2 heures après que le sujet d'essai ait développé un symptôme de maladie cardiaque.

3. Procédé selon la revendication 1 ou 2, dans lequel l'échantillon biologique est un échantillon de sang.

4. Procédé selon l'une quelconque des revendications 1 à 3, dans lequel, dans l'étape (b), les informations montrant que le sujet d'essai n'a pas un infarctus du myocarde aigu sont obtenues lorsque la valeur mesurée est inférieure au seuil.

5. Procédé selon l'une quelconque des revendications 1 à 4, dans lequel la valeur mesurée de peroxyrédoxine est un taux d'expression d'un gène de peroxyrédoxine ou une valeur mesurée d'une protéine de peroxyrédoxine.

6. Utilisation d'un réactif pour le procédé pour soutenir un diagnostic d'infarctus du myocarde aigu selon l'une quelconque des revendications 1 à 5, dans laquelle le réactif comprend un anticorps anti-peroxyrédoxine.

7. Utilisation d'une trousse pour le procédé pour soutenir un diagnostic d'infarctus du myocarde aigu selon l'une quelconque des revendications 1 à 5, la trousse comprenant :
un premier anticorps anti-peroxyrédoxine ;
une phase solide ;
un deuxième anticorps anti-peroxyrédoxine et une substance de marquage.
